# EUROPÄISCHE PATENTANMELDUNG

(11) **EP 1 215 189 A1**
(43) Veröffentlichungstag der Anmeldung: **19.06.2002**
(21) Anmeldenummer: 01128389.2
(22) Anmeldetag: 03.12.2001
(51) Int. Cl.: C07C 35/37, C07C 29/17, C07C 29/56, C11B 9/00

(54) **Verfahren zur Herstellung von Isolongifolanol**

(30) Priorität: 14.12.2000 DE 10062418; 12.02.2001 DE 10106421
(71) Anmelder: HAARMANN & REIMER GMBH, 37601 Holzminden (DE)
(72) Erfinder: Wöhrle, Ingo, Dr., 37603 Holzminden (DE); Nienhaus, Jürgen, Dr., 37603 Holzminden (DE)
(74) Vertreter: Mann, Volker, Dr.

(57) **Zusammenfassung**

Die Erfindung betrifft die Herstellung der Verbindungen Isolongifolanol (IUPAC-Name: 2,2,7,7-tetramethyltricyclo[6.2.1.0^{1,6}]undecan-6-ol) und Isolongifolenol (IUPAC-Name: 2,2,7,7-tetramethyltricyclo[6.2.1.0^{1,6}]undec-4-en-6-ol) sowie deren Verwendung als Riech- oder Aromastoff.

## Beschreibung

Die Erfindung betrifft die Herstellung der Verbindungen Isolongifolanol (IUPAC-Name: 2,2,7,7-Tetramethyltricyclo[6.2.1.0^{1,6}]undecan-6-ol) und Isolongifolenol (1,3,4,5,6,8a-Hexahydro-1,1,5,5-tetramethyl-2,4a-methanonaphthalin-8a(2H)-ol; ICTPAC-Name: 2,2,7,7-tetramethyltricyclo[6.2.1.0^{1,6}]undec-4-en-6-ol), sowie deren Verwendung als Riech- oder Aromastoffe in der funktionellen Parfümerie und in der Feinparfümerie.

Es besteht ein ständiger Bedarf an neuen Riechstoffen mit interessanten Duftnoten, besonders an solchen mit zusätzlichem Nutzen. Dieser Bedarf ergibt sich aus der notwendigen Anpassung an die wechselnden Trends und Moden und der damit einhergehenden Notwendigkeit der Ergänzung der bestehenden Palette an natürlichen Riechstoffen.

Des weiteren besteht generell ein ständiger Bedarf an synthetischen Riechstoffen, die sich günstig und mit gleichbleibender Qualität herstellen lassen. Diese Substanzen sollen möglichst angenehme und naturnahe Geruchsprofile aufweisen.

Aufgrund der stark schwankenden Preise und Qualitäten natürlicher Patchouli-Öle besteht ein dringender Bedarf an synthetischen Verbindungen mit Patchouli-Charakter und holzig-erdigen Noten, die in einer effizienten Synthese aus kostengünstigen Ausgangsprodukten herstellbar sind und darüber hinaus mit ihren originellen Dufteigenschaften die Kompositionsmöglichkeiten des Parfümeurs erweitern.

Aus JP 58/022450 B4 und JP 60/010007 B4 ist Isolongifolanol **(4)** bekannt. Die Verbindung wird in dem dort beschriebenen Verfahren jedoch nur mit einem Gehalt von 5% durch Hydrierung des Isolongifolenoxids **(2)** in einem Mehrkomponentengemisch erhalten. In J. Agric. Food. Chem. 1994, 42, 138-142 ist die Umsetzung von Isolongifolenoxid **(2)** mit Lithiumaluminiumhydrid beschrieben. Auf diese Weise wurden geringe Mengen (<100 mg) des Isolongifolanols **(4)** mit einer Ausbeute von 76% erhalten. Nachteilig an diesem Verfahren ist der Preis des Reduktionsmittels sowie die sicherheitstechnischen Probleme, die mit der Handhabung von Lithiumaluminiumhydrid einhergehen.

Es bestand daher weiterhin die Aufgabe Verbindung **4** in einer effizienten, im industriellen Maßstab realisierbaren, sicheren Synthese aus kostengünstigen Ausgangsprodukten herzustellen.

Die Erfindung betrifft daher ein Verfahren zur Herstellung von Isolongifolanol **(4)**, gekennzeichnet durch die folgenden Verfahrensschritte:
a) Epoxidierung von Isolongifolen **(1)** zu Isolongifolenoxid **(2)**,
b) Umlagerung von Isolongifolenoxid **(2)** in Gegenwart einer Base zu Isolongifolenol **(3)** und
c) Reduktion von Isolongifolenol **(3)** zu Isolongifolanol **(4)**.

Folgendes Formelschema kann die Erfindung erläutern:

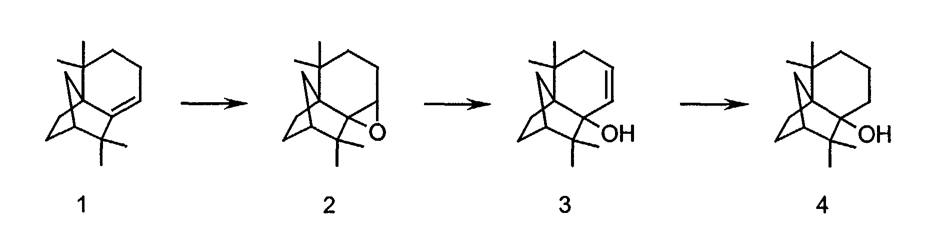

In der ersten Stufe kann in bekannter Weise Isolongifolen **(1)** beispielsweise mit Peressigsäure zu Isolongifolenoxid **(2)** umgesetzt werden (Tetrahedron Lett. 1964, 8, 417; J. Org. Chem. 1970, 35, 1172).

Im zweiten Schritt kann das Epoxid **2** in thermisch und chemisch inerten Lösungsmitteln in Anwesenheit einer starken Base zu Isolongifolenol **(3)** umgelagert werden.

Bedingungen, unter denen die Umlagerung günstigerweise durchgeführt werden kann sind beispielsweise zu finden in Helv. Chim. Acta 1967, 50, 153; Synthesis 1972, 194 oder auch Tetrahedron 1983, 39, 2323. Eine Übersicht ist gegeben in Larock, Comprehensive Organic Transformations, VCH, 1989, 117-118.

Starke Basen sind typischerweise Alkali-, Erdalkali- bzw. Leichtmetallalkoholate oder Alkali-, Erdalkali- bzw. Leichtmetallamide.

Leichtmetalle im Sinne dieser Erfindung sind insbesondere Aluminium, Titan und Beryllium.

Besonders überraschend ist, dass die Umlagerung mit unverzweigten Metall-alkoholaten wie Methanolaten bzw. Ethanolaten mit sehr hoher Selektivität zum Isolongifolenol führt. Mit diesen Basen entstehen typischerweise aus Epoxiden die korrespondierenden vicinalen Hydroxyalkylether (Chem. Rev. 1959, 737).

Als Basen seien beispielsweise genannt Lithiumdiethylamid, Lithium-n-dipropylamid, Lithiumdiisopropylamid, Lithium-n-dibutylamid, Lithiumethylendiamid, Trilithiumphosphat, Natriumhydrid, Kaliumhydrid, Diethylaluminium-2,2,6,6-tetramethylpiperidid, Kalium-tert-butanolat, Natrium-tert-butanolat, Lithium-tert-butanolat, Natriumethanolat, Kaliumethanolat, Lithiumethanolat, Natriummethanolat, Kaliummethanolat, Lithiummethanolat, Natriumisopropanolat, Kaliumisopropanolat, Lithiumisopropanolat, Magnesiumethanolat, Magnesiummethanolat, Calciumethanolat und Calciummethanolat.

Bevorzugte Basen sind Lithium-, Natrium- und Kaliumalkoholate mit 1 bis 6 Kohlenstoffatomen.

Insbesondere bevorzugt sind Natriummethanolat, Natriumethanolat, Natrium-isopropanolat, Natrium-tert-butanolat, Kaliummethanolat, Kaliumethanolat, Kalium-isopropanolat, Kalium-tert-butanolat, Lithiummethanolat, Lithiumethanolat, Lithium-isopropanolat und Lithium-tert-butanolat.

Es werden 0,3 bis 2,5 molare Äquivalente der Base eingesetzt, bevorzugt 0,6 bis 1,8 Äquivalente und insbesondere bevorzugt 0,8 bis 1,4 Äquivalente. Die Äquivalente beziehen sich hierbei auf den Gehalt an Epoxid **2**.

Die Reaktion kann in einer Vielzahl von Lösungsmitteln durchgeführt werden. Geeignet sind generell unpolare oder aprotisch polare Lösungsmittel.

Als Lösungsmittel seien offenkettige oder cyclische Dialkyl- bzw. Alkylarylether wie z.B: Diethylether, Tetrahydrofuran, Anisol, aliphatische oder aromatische Kohlenwasserstoffe mit 6 bis 10 Kohlenstoffatomen wie Cyclohexan, n-Heptan, Isooctan, Toluol, Ethylbenzol, Xylole, offenkettige oder cyclische N,N-Diniederalkylcarbonsäureamide wie z.B. N,N-Dimethylformamid, N,N-Dimethylacetamid, N-Methyl-2-pyrrolidinon oder Sulfoxide wie z.B. Dimethylsulfoxid und Diethylsulfoxid genannt.

Bevorzugte Lösungsmittel sind aromatische Kohlenwasserstoffe mit 7 bis 9 Kohlenstoffatomen, N,N-Dimethylformamid, N,N-Dimethylacetamid, N-Methyl-2-pyrrolidinon, N-Ethylpyrrolidinon, N-Methylvalerolactam, N-Methylcaprolactam, Dimethylsulfoxid, Xylole und Anisol.

Besonders bevorzugte Lösungsmittel sind N,N-Dimethylformamid, N-Methyl-2-pyrrolidinon, Dimethylsulfoxid, Xylole und Anisol.

Die Umlagerung kann im Temperaturbereich von 0 bis 250°C durchgeführt werden, bevorzugt im Bereich von 50 bis 220°C, insbesondere bevorzugt im Bereich von 80 bis 190°C.

Die Reaktion kann bei 0 bis 200 bar durchgeführt werden, bevorzugt sind Drücke im Bereich 1 bis 50 bar.

Die Reduktion von **3** zu **4** im dritten Schritt erfolgt vorteilhafterweise an Hydrierkatalysatoren in einer Wasserstoffatmosphäre. Als Hydrierkatalysatoren eignen sich beispielsweise Elemente der 8. Nebengruppe des Periodensystems. Besonders vorteilhaft sind hier die Elemente Nickel, Palladium, Platin, Rhodium, Iridium, Ruthenium sowie deren Mischungen, Verbindungen und Legierungen. Diese Katalysatoren können beispielsweise in fein verteilter Form, aufgebracht auf Träger oder zusammen mit anderen Metallen oder deren Verbindungen eingesetzt werden.

Als vorteilhafte Trägermaterialen seien genannt Aktivkohle, Aluminiumoxide, Metalloxide, Kieselgele, Zeolithe, Tone, Tongranulate, amorphe Aluminiumsilicate, oder sonstige anorganische oder polymere Träger.

Der Wasserstoffdruck bei der Hydrierungsreaktion liegt im Bereich von 1 bis 200 bar, bevorzugt im Bereich von 1 bis 100 bar, insbesondere bevorzugt im Bereich von 5 bis 50 bar.

Das Isolongifolanol **(4)** kann nach üblichen Methoden, z.B. durch Destillation oder Kristallisation, gereinigt werden.

Die nach dem erfindungsgemäßen Verfahren hergestellte Verbindung **4** ist ein Riechstoff und verfügt über komplexe Geruchseigenschaften. Neben Patchouli- und Holznoten weist sie kampherige, ambrierte sowie grüne Noten auf. Vorteilhaft ist die Einarbeitung in holzige und würzige Kompositionen, daneben gelingt mit **4** eine hervorragende Nachstellung natürlicher Patchouli-Öle.

Ein weiterer Teil der vorliegenden Erfindung betrifft Isolongifolenol der Strukturformel:

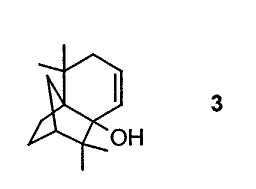

Die erfindungsgemäße Verbindung Isolongifolenol **(3)** verfügt über bemerkenswerte und komplexe Geruchseigenschaften. Neben den besonders gefragten Patchouli- und Holznoten weist sie ebenfalls kräftige erdig-kampherige sowie moosig-herbe Aspekte auf. Die Substanz zeichnet sich durch Originalität und natürlichen Charakter, sowie durch eine sehr hohe Intensität verbunden mit einer guten Haftfestigkeit aus.

Überraschenderweise unterscheidet sich Isolongifolenol **(3)** geruchlich deutlich vom Isolongifolanol **(4)** durch seinen sehr viel erdigeren Geruch und den intensiveren, Patchouli-typischen Charakter.

Das erfindungsgemäße Isolongifolenol **(3)** kann nach üblichen Methoden, z.B. durch Destillation oder Kristallisation, gereinigt werden.

Durch die Verwendung des erfindungsgemäßen Isolongifolenols **(3)** lässt sich in der Regel bereits in geringer Dosierung in den resultierenden Riechstoffkompositionen feine, holzige-erdige Patchoulinoten erzielen, wobei der geruchliche Gesamteindruck auffallend harmonisiert. Besonders effektiv ist die Einarbeitung in holzige, Chypre, orientalische und würzige Kompositionen. In höherer Dosierung gelingt eine hervorragende Nachstellung natürlicher Patchouli-Öle.

Das erfindungsgemäße Isolongifolenol **(3)** kann dabei als Einzelstoff in einer Vielzahl von Produkten verwendet werden; besonders vorteilhaft lässt es sich mit anderen Riechstoffen zu neuartigen Riechstoffkompositionen kombinieren.

Beispiele für Riechstoffe, mit denen das erfindungsgemäße Isolongifolenol **(3)** wie auch das daraus herstellbare Isolongifolanol **(4)** vorteilhaft kombiniert werden können, finden sich z.B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 3^{rd}. Ed., Wiley-VCH, Weinheim 1997.

### Im einzelnen seien genannt:

Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;

Einzel-Riechstoffe aus der Gruppe der Kohlenwasserstoffe, wie z. B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecadiensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinoat; Methyl-2-noninoat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavandulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpineol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,25,E9-cyclododecatrien-1-ol; 2-Isobutyl-4-methyltetrahydro-2H-pyran-4-ol;
der cycloaliphatischen Alkohole wie z.B. alpha-3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 5-Cyclohexadecen-1-on; 8-Cyclohexadecen-1-on; 9-Cycloheptadecen-1-on; Cyclopentadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1 -Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)-propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methyl-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cisund trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die das erfindungsgemäße Isolongifolenol enthaltenden Parfümöle können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Des weiteren können die das erfindungsgemäße Isolongifolenol **(3)** enthaltenden Parfümöle an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die das erfindungsgemäße Isolongifolenol enthaltenden Parfümöle können auch mikroverkapselt, sprühgetrocknet, als Einschluß-Komplexe oder als Extrusions-Produkte vorliegen und in dieser Form dem zu parfümierenden Produkt hinzugefügt werden.

Gegebenenfalls können die Eigenschaften der derart modifizierten Parfümöle durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erhalten werden.

In den erfindungsgemäßen Riechstoffkompositionen beträgt die eingesetzte Menge des erfindungsgemäßen Isolongifolenols **(3)** 0,05 bis 80 Gew.-%, vorzugsweise 0,5 bis 50 Gew.-%, bezogen auf das gesamte Parfümöl.

Die das erfindungsgemäße Isolongifolenol enthaltenden Parfümöle können in konzentrierter Form, in Lösungen oder in oben beschriebener modifizierter Form verwendet werden für die Herstellung von z.B. Parfüm-Extraits, Eau de Parfüms, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungs-mitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und - lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigende Haarlotionen, Haarspülungen, permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Als besonders gut stellte sich die Einarbeitung des erfindungsgemäßen Isolongifolenols in Parfümöle für Shampoos und Duschgels heraus. Ein weiterer Schwerpunkt der Verwendung des erfindungsgemäßen Isolongifolenols liegt wegen seiner Stabilität im alkalischen Bereich bei der Parfümierung von Seifen und Waschmitteln. Bei der Verwendung in Waschmittelparfümierungen zeichnet sich das erfindungsgemäße Isolongifolenol durch seine Natürlichkeit und seine hohe Substantivität, d.h. seine gute Haftung auf der gewaschenen Faser, aus. Dies gilt ebenfalls für Isolongifolanol.

Das erfindungsgemäße Verbindung Isolongifolenol weist neben ihrer Medienstabilität (in sauren, basischen und oxidativen Medien) die Eigenschaft auf, den Geruchseindruck anderer Verbindungen zu verstärken, d.h sie ist ein sogenannter Enhancer oder Booster.

Als zusätzlich bemerkenswerte Eigenschaft des Isolongifolenols ist der mit dieser Verbindung erzielbare geruchsmaskierende oder geruchsüberdeckende Effekt zu nennen, der insbesondere im Bereich der Körper- und Haarpflegeprodukte von Interesse ist. Ähnliche Effekte können mit Isolongifolanol erzielt werden.

### Beispiele

Die folgenden Beispiele erläutern die Erfindung:

Die in den Beispielen angegebenen Prozentangaben beziehen sich auf Gewichtsprozente. Ausgehend von Isolongifolen (GC-Reinheit: etwa 78%) wurde nach Literaturangaben (s.o.) mittels Peressigsäure-Oxidation das Isolongifolenepoxid (2) in einer GC-Reinheit von etwa 75% erhalten, welches in den folgenden Beispielen zum Einsatz kommt.

### Beispiel 1

In einer Schutzgasatmosphäre wurden 333 g Isolongifolenepoxid (1.1 mol bezogen auf den GC-Gehalt an Epoxid) in 500 g Dimethylsulfoxid gelöst. Hierzu wurden 123 g (1.1 mol) Kalium-tert-butanolat zugegeben und das Gemisch 12 Stunden auf 95°C erhitzt. Nach beendeter Reaktion (vollständiger Umsatz) wurde das Reaktionsgemisch auf 400 ml Wasser gegeben, die Phasen getrennt und die wässrige Phase viermal mit n-Heptan extrahiert. Die vereinigten organischen Phasen wurden mit Wasser gewaschen und mit Natriumsulfat getrocknet. Das nach Entfernung des Lösungsmittels verbleibende Öl wurde zur weiteren Reinigung eingesetzt.

Der Rückstand wurde über eine mit Stahlwendeln oder Glasringen gefüllte Kolonne fraktioniert, wobei 242 g Isolongifolenol bei 100-105°C / 1 mbar mit einer GC-Reinheit >93% erhalten werden.

Durch Umkristallisation aus n-Hexan oder Ethanol kann die Substanz weiter gereinigt werden. Schmelzpunkt: 37-38°C (n-Hexan).
¹H-NMR (400 MHz, CDCl₃): δ (ppm) = 0.92 (q, J = 0.5 Hz, 3H), 0.95 (m, 6H), 0.98 (m, 3H), 1.16 (s, 1H), 1.25 (dd, J = 1.8, 10.0 Hz, 1H), 1.39 (m, 2H), 1.57 (ddt, J = 1.9, 2.8, 10.0 Hz, 1H), 1.64 (m, 1H), 1.74 (ddd, J = 0.9, 6.0, 17.6 Hz, 1H), 1.81 (m, 2H), 2.01 (dsept, J = 0.9, 17.6 Hz, 1H), 5.58 (ddd, J = 1.9, 6.0, 10.3 Hz, 1H), 5.67 (ddt, J = 0.9, 2.8, 10.3, 1H).
¹³C-NMR (100 MHz, CDCl₃): δ (ppm) = 20.61, 21.73, 24.37, 25.29, 26.80, 27.53, 32.10, 35.90, 39.02, 44.08, 47.77, 58.24, 77.82, 124.43, 130.88.

### Beispiel 2

In einer Schutzgasatmosphäre wurden 320 g Isolongifolenepoxid (1.06 mol bezogen auf den GC-Gehalt an Epoxid) in 300 g N-Methyl-2-pyrrolidinon gelöst. Hierzu wurden 88.5 g (1.3 mol) Natriumethanolat zugegeben und das Gemisch 10 Stunden auf 160°C erhitzt. Nach beendeter Reaktion (Umsatz: 95%) wurde das Lösungsmittel im Vakuum weitgehend abdestilliert und der Rückstand auf 300 ml Wasser und 150 g Toluol verteilt. Nach Abtrennung der wässrigen Phase wurde diese noch zweimal mit Toluol extrahiert und die vereinigten organischen Phasen mit Wasser gewaschen. Die Produktphase wurde mit Natriumsulfat getrocknet und eingeengt. Die weitere Reinigung des Rohproduktes kann wie in Beispiel 1 erfolgen. Nach Destillation wurden 215 g Isolongifolenol erhalten (GC-Reinheit: 92%).

### Beispiel 3

In einer Schutzgasatmosphäre wurden 333 g Isolongifolenepoxid (1.1 mol bezogen auf den GC-Gehalt an Epoxid) in 500 g N,N-Dimethylformamid gelöst. Hierzu wurden 97 g (1.8 mol) Natriummethanolat zugegeben und das Gemisch 18 Stunden auf 180°C erhitzt. Nach beendeter Reaktion (Umsatz: 93%) erfolgte die Aufarbeitung wie in Beispiel 2 beschrieben. Nach Destillation wurden 213 g Isolongifolenol erhalten (GC-Reinheit: >94%).

### Beispiel 4

In einer Schutzgasatmosphäre wurden 333 g Isolongifolenepoxid (1.1 mol bezogen auf den GC-Gehalt an Epoxid) in 500 g Anisol gelöst. Hierzu wurden 141 g (1.25 mol) Kalium-tert-butanolat zugegeben und das Gemisch 16 Stunden auf 140°C erhitzt. Nach beendeter Reaktion (Umsatz: 92%) wurde das Reaktionsgemisch auf 250 ml Wasser gegeben, die Phasen getrennt und die wässrige Phase zweimal mit Toluol extrahiert. Die vereinigten organischen Phasen wurden mit Wasser gewaschen, mit Natriumsulfat getrocknet und eingeengt. Die Destillation des Rückstandes ergab 220 g Isolongifolenol (GC-Reinheit: 94%).

### Beispiel 5

In einer Schutzgasatmosphäre wurden 73.2 g Diethylamin (1 mol) in 300 ml Diethylether vorgelegt und mittels eines Eisbades auf 0°C abgekühlt. Hierzu wurden innerhalb von 30 Minuten 42 ml einer 2.5 M Lösung von n-Butyllithium in Hexan zugetropft. Nach 10 Minuten wurden 272 g Isolongifolenepoxid (0.9 mol bezogen auf den GC-Gehalt an Epoxid) in 300 ml Diethylether innerhalb einer Stunde zugetropft. Nach beendeter Zugabe wurde das Kühlbad entfernt und das Reaktionsgemisch 16 Stunden unter Rückfluss erhitzt. Die abgekühlte Reaktionsmischung wurde auf 500 g Eis gegossen. Nach Phasentrennung wurde die wässrige Phase dreimal mit Diethylether extrahiert. Die vereinigten organischen Phasen wurden nacheinander mit 1N Salzsäure, Bicarbonat-Lösung und Wasser gewaschen. Nach Trocknen mit Natriumsulfat wurde das Lösungsmittel entfernt. Die Destillation des Rückstandes ergab 155 g Isolongifolenol (GC-Reinheit: 94%).

### Beispiel 6

In einer Schutzgasatmosphäre wurden 333 g Isolongifolenepoxid (1.1 mol bezogen auf den GC-Gehalt an Epoxid) und 11.6 g (0.1 mol) Trilithiumphosphat zusammengegeben. Das Gemisch wurde 4 Stunden auf 160°C erhitzt. Die abgekühlte Reaktionsmischung (Umsatz: 84%) wurde auf 100 ml Wasser und 150 ml Diethylether gegeben, die Phasen getrennt und die wässrige Phase zweimal mit Diethylether extrahiert. Die vereinigten organischen Phasen wurden mit Wasser gewaschen, mit Natriumsulfat getrocknet und vom Lösungsmittel befreit. Die destillative Aufreinigung des Rückstandes ergab 138 g Isolongifolenol (GC-Reinheit: 95%).

### Beispiel 7

In einer Schutzgasatmosphäre wurden 7 g Lithium in 300 ml wasserfreiem Ethylendiamin aufgelöst. Bei 110°C wurden nun langsam 151 g Isolongifolenepoxid (0.5 mol bezogen auf den GC-Gehalt an Epoxid) zugetropft und die Mischung weitere 3 Stunden bei dieser Temperatur gerührt. Zu der abgekühlten Reaktionsmischung wurden vorsichtig 200 ml Wasser gegeben, die Phasen getrennt und die wässrige Phase zweimal mit Diethylether extrahiert. Die vereinigten organischen Phasen wurden mit Wasser gewaschen, mit Natriumsulfat getrocknet und eingeengt. Die destillative Aufreinigung des Rückstandes ergab 78 g Isolongifolenol (GC-Reinheit: >92%).

### Beispiel 8

In einer Schutzgasatmosphäre wurden 333 g Isolongifolenepoxid (1.1 mol bezogen auf den GC-Gehalt an Epoxid) in 500 g p-Xylol gelöst. Hierzu wurden 132 g (1.18 mol) Kalium-tert-butanolat zugegeben und das Gemisch 24 Stunden auf Rückflußtemperatur erhitzt. Nach beendeter Reaktion (Umsatz: 92%) wurde das Reaktionsgemisch auf 200 ml Wasser gegeben, die Phasen getrennt und die wässrige Phase zweimal mit Toluol extrahiert. Die vereinigten organischen Phasen wurden mit Wasser gewaschen, mit Natriumsulfat getrocknet und eingeengt. Die Destillation des Rückstandes ergab 216 g Isolongifolenol (GC-Reinheit: 94%).

### Beispiel 9

Anhand des vorliegenden Beispiels kann die Nachstellung eines Parfümöls mit natürlichem Patchouli-Charakter verdeutlicht werden.

| Ingredienzien | Gewichtsteile |
|---|---|
| 1. Isobornylacetat | 5,5 |
| 2. Borneol L | 25,0 |
| 3. Kampfer | 15,0 |
| 4. Davanaöl f. Parf. | 1,0 |
| 5. Nopol | 95,0 |
| 6. Dimethylbenzylcarbinylacetat | 5,5 |
| 7. Terpineol rein | 11,0 |
| 8. Palmarosaöl | 6,0 |
| 9. Caryophyllen rekt. | 20,0 |
| 10. Butylcyclohexanol para-tert | 32,5 |
| 11. Cedernholzöl Florida | 25,0 |
| 12. Palisandin (H&R) | 162,0 |
| 13. Vetiveröl Java | 5,5 |
| 14. Gurjunbalsam Siam | 102,5 |
| 15. Sandel 80 (H&R) | 7,5 |
| 16. Fixateur Bois | 35,0 |
| 17. Elemi Resin EE | 36,5 |
| 18. Opoponax Resin entwachst | 5,0 |
| 19. Karanal (Quest) | 2,5 |
| 20. Globalid 100% (H&R) | 2,0 |
| 21. Isolongifolenol | 400,0 |

### Beispiel 10

In einer Hydrierapparatur werden 200 g Isolongifolenol in 200 g Ethanol vorgelegt, 1% Hydrierkatalysator zugegeben (z.B. 5% Palladium auf Aktivkohle) und bei 10 bar Wasserstoffdruck so lange hydriert, bis keine Wasserstoffaufnahme mehr zu verzeichnen ist. Nach Entfernen des Katalysators und des Lösungsmittels, wurde das Reaktionsprodukt gaschromatographisch analysiert. Der Umsatz an **3** lag bei >98%. Es wurden 197 g an Hydrierungsrohprodukt isoliert. Zur Reinigung des Isolongifolanols **4** wurde das Hydrierungsrohprodukt aus Ethanol oder Hexan bzw. Petrolether umkristallisiert.

Das so erhaltene Isolongifolanol weist eine Reinheit >99% auf und besitzt einen Schmelzpunkt von 67°C (n-Hexan).

## Patentansprüche

1. Verfahren zur Herstellung von Isolongifolanol **(4)**, **gekennzeichnet durch** die folgenden Verfahrensschritte:
a) Epoxidierung von Isolongifolen **(1)** zu Isolongifolenoxid **(2)**,
b) Umlagerung von Isolongifolenoxid **(2)** in Gegenwart einer Base zu Isolongifolenol **(3)** und
c) Reduktion von Isolongifolenol **(3)** zu Isolongifolanol **(4)**.

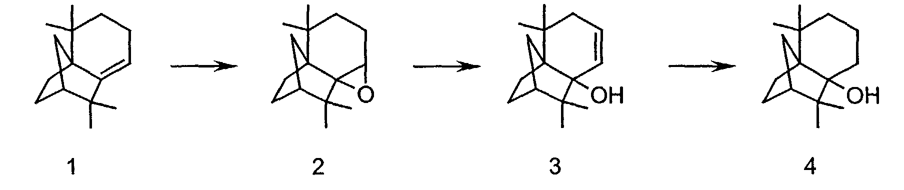

2. Verfahren nach Anspruch 1, **dadurch gekennzeichnet, dass** im Verfahrensschritt b) als Base Alkali-, Erdalkali- bzw. Leichtmetallalkoholate oder Alkali-, Erdalkali- bzw. Leichtmetallamide eingesetzt werden.

3. Verfahren nach Anspruch 1, **dadurch gekennzeichnet, dass** im Verfahrensschritt b) als Base Natriummethanolat, Natriumethanolat, Natrium-isopropanolat, Natrium-tert-butanolat, Kaliummethanolat, Kaliumethanolat, Kalium-isopropanolat, Kalium-tert-butanolat, Lithiummethanolat, Lithiumethanolat, Lithium-isopropanolat oder Lithium-tert-butanolat eingesetzt werden.

4. Verfahren nach Anspruch 1, **dadurch gekennzeichnet, dass** der Verfahrensschritt b) im Temperaturbereich von 0 bis 250°C durchgeführt wird.

5. Isolongifolenol der Formel

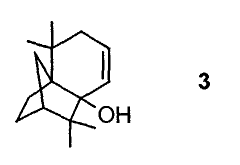

6. Verwendung von Isolongifolenol als Riech- oder Aromastoff.

7. Riechstoffkompositionen, enthaltend Isolongifolenol.

8. Verwendung von Isolongifolanol als Riech- oder Aromastoff.
